# EUROPEAN PATENT SPECIFICATION

(11) **EP 4 032 528 B1**
(45) Date of publication and mention of the grant of the patent: **08.04.2026**
(21) Application number: 22152747.6
(22) Date of filing: 21.01.2022
(51) Int. Cl.: A61K 9/127, A61K 31/445, A61P 1/00, A61K 9/1277

(54) **MANUFACTURING OF BUPIVACAINE MULTIVESICULAR LIPOSOMES**
HERSTELLUNG VON MULTIVESIKULÄREN BUPIVACAIN-LIPOSOMEN
FABRICATION DE LIPOSOMES MULTIVÉSICULAIRES DE BUPIVACAÏNE

(30) Priority: 22.01.2021 US 202117156400; 22.01.2021 US 202117156424
(43) Date of publication of application: 27.07.2022
(60) Divisional application: 26154324.3
(73) Proprietor: Pacira Pharmaceuticals, Inc., San Diego, CA 92121 (US)
(72) Inventor: HALL, Jeffrey S., San Diego (US); TURNBULL, David J., San Diego (US); GRIGSBY Jr., John J., San Diego (US); ARDEKANI, Soroush M., San Diego (US); DAVIS, Paige N., San Diego (US); GARCIA, Louie D., San Diego (US); KURZ, Stephanie M., San Diego (US); LOS, Kathleen D. A., San Diego (US)
(74) Representative: Bassil, Nicholas Charles

(56) References cited:
- CN-A- 110 179 752
- CN-B- 108 078 929
- US-A1- 2011 250 264

## Description

### BACKGROUND

### Field

This disclosure relates generally to commercial manufacturing processes for making bupivacaine multivesicular liposomes using independently operating tangential flow filtration systems.

### Description of the Related Art

Bupivacaine is a versatile drug that has been shown to be efficacious for a wide variety of indications, including: local infiltration, peripheral nerve block, sympathetic nerve block, and epidural and caudal blocks. It may be used in pre-, intra- and post-operative care settings. Bupivacaine encapsulated multivesicular liposomes (Exparel^{®}) has been approved in the US and Europe for use as postsurgical local analgesia and as an interscalene brachial plexus nerve block to produce postsurgical regional analgesia, providing significant long-lasting pain management across various surgical procedures. Particularly, Exparel^{®} has had great success in the market in part due to the ability to locally administer bupivacaine multivesicular liposomes (MVLs) at the time of surgery and extend the analgesic effects relative to other non-liposomal formulations of bupivacaine. Such extended release properties of bupivacaine MVLs allow patients to control their post-operative pain without or with decreased use of opioids. Given the addictive nature of opioids and the opioid epidemic that has been affecting countries around the world, there is an urgent need for new and improved large scale productions of Exparel^{®} to meet the substantial and growing market demand. Patent document CN110179752 discloses a process for preparing a composition of bupivacaine encapsulated multivesicular liposomes (MVLs).

### SUMMARY

Some aspects of the present disclosure relate to a crossflow filtration system comprising:
a diafiltration vessel; and
a plurality of independently operating crossflow modules, each crossflow module of the plurality of independently operating crossflow modules comprising at least one filter array, each filter array comprising a plurality of hollow fiber filters, wherein each crossflow module of the plurality of independently operating crossflow modules is connected to a retentate conduit, a permeate conduit, and a rotary lobe pump. In some embodiments, the crossflow filtration system may be used in the microfiltration and/or diafiltration step of the commercial process described herein. The scope of the present invention is defined in the claims.

Some aspects of the present disclosure relate to a process for preparing bupivacaine encapsulated multivesicular liposomes in a commercial scale, the process comprising:
(a) mixing a first aqueous solution comprising phosphoric acid with a volatile water-immiscible solvent solution to form a water-in-oil first emulsion, wherein the volatile water-immiscible solvent solution comprises bupivacaine, at least one amphipathic lipid and at least one neutral lipid;
(b) mixing the water-in-oil first emulsion with a second aqueous solution to form a water-in-oil-in-water second emulsion;
(c) removing the volatile water-immiscible solvent from the water-in-oil-in-water second emulsion to form a first aqueous suspension of bupivacaine encapsulated multivesicular liposomes having a first volume;
(d) reducing the first volume of the first aqueous suspension of bupivacaine encapsulated multivesicular liposomes by microfiltration to provide a second aqueous suspension of bupivacaine encapsulated multivesicular liposomes having a second volume;
(e) exchanging the aqueous supernatant of the second aqueous suspension with a saline solution by diafiltration to provide a third aqueous suspension of bupivacaine encapsulated multivesicular liposomes having a third volume; and
(f) further reducing the third volume of the third aqueous suspension by microfiltration to provide a final aqueous suspension of bupivacaine encapsulated multivesicular liposomes having a target concentration of bupivacaine;
wherein all steps are carried out under aseptic conditions.

Some aspects of the present disclosure as claimed relate to a composition of bupivacaine encapsulated multivesicular liposomes (MVLs) prepared by a commercial scale process, the commercial scale process comprising:
(a) mixing a first aqueous solution comprising phosphoric acid with a volatile water-immiscible solvent solution to form a water-in-oil first emulsion, wherein the volatile water-immiscible solvent solution comprises bupivacaine, 1, 2-dierucoylphosphatidylcholine (DEPC), 1, 2-dipalmitoyl-sn-glycero-3 phospho-rac-(1-glycerol) (DPPG), and at least one neutral lipid;
(b) mixing the water-in-oil first emulsion with a second aqueous solution to form a water-in-oil-in-water second emulsion;
(c) removing the volatile water-immiscible solvent from the water-in-oil-in-water second emulsion to form a first aqueous suspension of bupivacaine encapsulated MVLs having a first volume;
(d) reducing the first volume of the first aqueous suspension of bupivacaine encapsulated MVLs by microfiltration to provide a second aqueous suspension of bupivacaine encapsulated MVLs having a second volume;
(e) exchanging the aqueous supernatant of the second aqueous suspension with a saline solution by diafiltration to provide a third aqueous suspension of bupivacaine encapsulated MVLs having a third volume; and
(f) further reducing the third volume of the third aqueous suspension by microfiltration to provide a final aqueous suspension of bupivacaine encapsulated MVLs having a target concentration of bupivacaine;

wherein all steps are carried out under aseptic conditions; and
wherein the erucic acid concentration in the composition is 23 µg/mL or less after the composition is stored at 25°C for one month and less than 109 µg/mL after the final aqueous suspension is stored at 25°C for six months; and wherein the encapsulated lysine concentration in the final aqueous suspension of bupivacaine encapsulated multivesicular liposomes is at least 0.03 mg/mL.

Some aspect of the present disclosure relates to a composition of bupivacaine encapsulated multivesicular liposomes (MVLs), comprising: bupivacaine residing inside a plurality of internal aqueous chambers of the MVLs separated by lipid membranes, wherein the lipid membranes comprise 1, 2-dierucoylphosphatidylcholine (DEPC), 1, 2-dipalmitoyl-sn-glycero-3 phospho-rac-(1-glycerol) (DPPG), and at least one neutral lipid; and an aqueous medium in which the bupivacaine encapsulated MVLs are suspended; wherein the composition has an initial pH of about 7.0 to about 7.4, and wherein erucic acid concentration in the composition is 23 µg/mL or less after the composition is stored at 25°C for one month

Some additional aspect of the present disclosure relates to a composition of bupivacaine encapsulated multivesicular liposomes (MVLs) prepared by a commercial scale process, the commercial scale process comprising:
(a) mixing a first aqueous solution comprising phosphoric acid with a volatile water-immiscible solvent solution to form a water-in-oil first emulsion, wherein the volatile water-immiscible solvent solution comprises bupivacaine, 1, 2-dierucoylphosphatidylcholine (DEPC), 1, 2-dipalmitoyl-sn-glycero-3 phospho-rac-(1-glycerol) (DPPG), and at least one neutral lipid;
(b) mixing the water-in-oil first emulsion with a second aqueous solution to form a water-in-oil-in-water second emulsion, wherein the second aqueous solution comprises lysine and dextrose;
(c) removing the volatile water-immiscible solvent from the water-in-oil-in-water second emulsion to form a first aqueous suspension of bupivacaine encapsulated MVLs having a first volume;
(d) reducing the first volume of the first aqueous suspension of bupivacaine encapsulated MVLs by microfiltration to provide a second aqueous suspension of bupivacaine encapsulated MVLs having a second volume;
(e) exchanging the aqueous supernatant of the second aqueous suspension with a saline solution by diafiltration to provide a third aqueous suspension of bupivacaine encapsulated MVLs having a third volume; and
(f) further reducing the third volume of the third aqueous suspension by microfiltration to provide a final aqueous suspension of bupivacaine encapsulated MVLs having a target concentration of bupivacaine;

wherein all steps are carried out under aseptic conditions; and
wherein the internal pH of the bupivacaine encapsulated MVLs in the composition is about 5.50. In some embodiments, the internal pH is measured after the composition has been stored at about 2-8 °C for at least 3 months, 6 months or 9 months.

Some additional aspect of the present disclosure relates to a composition of bupivacaine encapsulated multivesicular liposomes (MVLs), comprising: bupivacaine residing inside a plurality of internal aqueous chambers of the MVLs separated by lipid membranes, wherein the lipid membranes comprise 1, 2-dierucoylphosphatidylcholine (DEPC), 1, 2-dipalmitoyl-sn-glycero-3 phospho-rac-(1-glycerol) (DPPG), and at least one neutral lipid; and an aqueous medium in which the bupivacaine encapsulated MVLs are suspended; wherein the internal pH of the bupivacaine encapsulated MVLs is about 5.50.

In any aspects of the disclosure described herein, the composition of bupivacaine MVLs is suitable for human administration without further purification.

### BRIEF DESCRIPTION OF THE DRAWINGS

In addition to the features described above, additional features and variations will be readily apparent from the following descriptions of the drawings and exemplary embodiments. It is to be understood that these drawings depict typical embodiments, and are not intended to be limiting in scope.
FIG. 1A illustrates a process flow chart of the formation of an initial aqueous suspension bupivacaine MVLs according to an embodiment of the manufacturing process described herein.
FIG. 1B illustrates a process flow chart of additional steps of concentration, filtration and solvent removal of the initial aqueous suspension of bupivacaine MVLs according to an embodiment of the manufacturing process described herein.
FIG. 2 illustrate a crossflow filtration system according an embodiment of the manufacturing process described herein.
FIG. 3A is a line chart showing supernatant pH as a function of incubation time at 25°C for bupivacaine-MVL compositions prepared according to a manufacturing process described herein as compared to bupivacaine-MVL compositions using the existing manufacturing process.
FIG. 3B is a line chart showing erucic acid concentration as a function of incubation time at 25°C for bupivacaine-MVL compositions prepared according to a manufacturing process described herein as compared to bupivacaine-MVL compositions prepared by the existing commercial manufacturing process.
FIG. 3C is a chart showing erucic acid concentration as a function of supernatant pH at 25°C for bupivacaine-MVL compositions prepared according to a manufacturing process described herein as compared to bupivacaine-MVL compositions prepared by the existing commercial manufacturing process.

### DETAILED DESCRIPTION

Embodiments of the present disclosure relate to new and improved commercial scale manufacturing processes for making bupivacaine encapsulated multivesicular liposomes (MVLs). The newly developed processes provide up to 5 folds increase in final product volume as compared to the current process used for the manufacturing of Exparel^{®}, which is disclosed in U.S. Patent No. 9,585,838. The processes also allow for improved product operability. In addition, the improved and scaled up process also yields a more stabilized form of bupivacaine encapsulated MVLs, having less lipid degradation byproducts, increased internal pH, and increased lysine and dextrose encapsulation.

### Definitions

The section headings used herein are for organizational purposes only and are not to be construed as limiting the subject matter described.

As used herein, the terms "bupivacaine encapsulated multivesicular liposomes", "bupivacaine-MVLs" or "bupivacaine MVLs" refer to a multivesicular liposome composition encapsulating bupivacaine. In some embodiments, the composition is a pharmaceutical formulation, where the bupivacaine encapsulated multivesicular liposome particles are suspended in a liquid suspending medium to form a suspension. In some such embodiments, the BUP-MVL suspension may also include free or unencapsulated bupivacaine. In some cases, the free or unencapsulated bupivacaine may be less than about 8%, 7%, 6%, 5%, 4%, 3%, 2%, 1%, 0.5%, 0.2% or 0.1%, by weight of the total amount of the bupivacaine in the composition, or in a range defined by any of the two preceding values. In some embodiment, the free bupivacaine may be about 5% or less by weight of the total amount of the bupivacaine in the composition. In further embodiments, the free bupivacaine may be about 8% or less during the shelf life of the product (i.e., up to 2 years when stored at 2-8 °C).

As used herein, the term "encapsulated" means that bupivacaine is inside a liposomal particle, for example, the MVL particles, In some instances, bupivacaine may also be on an inner surface, or intercalated in a membrane, of the MVLs.

As used herein, the term "unencapsulated bupivacaine" or "free bupivacaine" refers to bupivacaine outside the liposomal particles, for example the MVL particles. For example, unencapsulated bupivacaine may reside in the suspending solution of these particles.

As used herein, the term "median particle diameter" refers to volume weighted median particle diameter of a suspension.

As used herein, a "pH adjusting agent" refers to a compound that is capable of modulating the pH of an aqueous phase.

As used herein, the terms "tonicity" and "osmolality" are measures of the osmotic pressure of two solutions, for example, a test sample and water separated by a semi-permeable membrane. Osmotic pressure is the pressure that must be applied to a solution to prevent the inward flow of water across a semi-permeable membrane. Osmotic pressure and tonicity are influenced only by solutes that cannot readily cross the membrane, as only these exert an osmotic pressure. Solutes able to freely cross the membrane do not affect tonicity because they will become equal concentrations on both sides of the membrane. An osmotic pressure provided herein is as measured on a standard laboratory vapor pressure or freezing point osmometer.

As used herein, the term "sugar" as used herein denotes a monosaccharide or an oligosaccharide. A monosaccharide is a monomeric carbohydrate which is not hydrolysable by acids, including simple sugars and their derivatives, e.g. aminosugars. Examples of monosaccharides include sorbitol, glucose, fructose, galactose, mannose, sorbose, ribose, deoxyribose, dextrose, neuraminic acid. An oligosaccharide is a carbohydrate consisting of more than one monomeric saccharide unit connected via glycosidic bond(s) either branched or in a chain. The monomeric saccharide units within an oligosaccharide can be the same or different. Depending on the number of monomeric saccharide units the oligosaccharide is a di-, tri-, tetra-, penta- and so forth saccharide. In contrast to polysaccharides, the monosaccharides and oligosaccharides are water soluble. Examples of oligosaccharides include sucrose, trehalose, lactose, maltose and raffinose.

As used herein, the term "amphipathic lipids" include those having a net negative charge, a net positive charge, and zwitterionic lipids (having no net charge at their isoelectric point).

As used herein, the term "neutral lipid" refers to oils or fats that have no vesicle-forming capabilities by themselves, and lack a charged or hydrophilic "head" group. Examples of neutral lipids include, but are not limited to, glycerol esters, glycol esters, tocopherol esters, sterol esters which lack a charged or hydrophilic "head" group, and alkanes and squalenes.

Unless defined otherwise, all technical and scientific terms used herein have the same meaning as is commonly understood by one of ordinary skill in the art. In the event that there are a plurality of definitions for a term herein, those in this section prevail unless stated otherwise. As used in the specification and the appended claims, the singular forms "a," "an" and "the" include plural referents unless the context clearly dictates otherwise. Unless otherwise indicated, conventional methods of mass spectroscopy, NMR, HPLC, protein chemistry, biochemistry, recombinant DNA techniques and pharmacology are employed. The use of "or" or "and" means "and/or" unless stated otherwise. Furthermore, use of the term "including" as well as other forms, such as "include", "includes," and "included," is not limiting. As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least." When used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

### Manufacturing Processes

Some embodiments of the present application relate to a commercial scale manufacturing process according to the claims for preparing bupivacaine encapsulated multivesicular liposomes. The process comprising:
(a) mixing a first aqueous solution comprising phosphoric acid with a volatile water-immiscible solvent solution to form a water-in-oil first emulsion, wherein the volatile water-immiscible solvent solution comprises bupivacaine, at least one amphipathic lipid and at least one neutral lipid;
(b) mixing the water-in-oil first emulsion with a second aqueous solution to form a water-in-oil-in-water second emulsion;
(c) removing the volatile water-immiscible solvent from the water-in-oil-in-water second emulsion to form a first aqueous suspension of bupivacaine encapsulated multivesicular liposomes having a first volume;
(d) reducing the first volume of the first aqueous suspension of bupivacaine encapsulated multivesicular liposomes by microfiltration to provide a second aqueous suspension of bupivacaine encapsulated multivesicular liposomes having a second volume;
(e) exchanging the aqueous supernatant of the second aqueous suspension with a saline solution by diafiltration to provide a third aqueous suspension of bupivacaine encapsulated multivesicular liposomes having a third volume; and
(f) further reducing the third volume of the third aqueous suspension by microfiltration to provide a final aqueous suspension of bupivacaine encapsulated multivesicular liposomes having a target concentration of bupivacaine;
wherein all steps are carried out under aseptic conditions.

In some embodiments of the process, the amphipathic lipid in the volatile water-immiscible solvent solution may be chosen from a wide range of lipids having a hydrophobic region and a hydrophilic region in the same molecule. Suitable amphipathic lipids include, but are not limited to zwitterionic phospholipids, including phosphatidylcholines, phosphatidylethanolamines, sphingomyelins, lysophosphatidylcholines, and lysophosphatidylethanolamines; anionic amphipathic phospholipids such as phosphatidylglycerols, phosphatidylserines, phosphatidylinositols, phosphatidic acids, and cardiolipins; cationic amphipathic lipids such as acyl trimethylammonium propanes, diacyl dimethylammonium propanes, stearylamine, and the like. Non-limiting exemplary phosphatidyl cholines include dioleyl phosphatidyl choline (DOPC), 1,2-dierucoyl phosphatidylcholine or 1,2-dierucoyl-sn-glycero-3-phosphocholine (DEPC), 1,2-didecanoyl-sn-glycero-3-phosphocholine (DDPC), 1,2-dilinoleoyl-sn-glycero-3-phosphocholine (DLOPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1-myristoyl-2-palmitoyl-sn-glycero 3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-oleoyl-sn-glycero-3-phosphocholine (POPC), 1-palmitoyl-2-stearoyl-sn-glycero-3-phosphocholine (PSPC), 1-stearoyl-2-myristoyl-sn-glycero-3-phosphocholine (SMPC), 1-stearoyl-2-oleoyl-sn-glycero-3-phosphocholine (SOPC), or 1-stearoyl-2-palmitoyl-sn-glycero-3-phosphocholine (SPPC). Non-limiting examples of phosphatidyl glycerols include dipalmitoylphosphatidylglycerol or 1,2-dipalmitoyl-sn-glycero-3-phospho-rac-(1-glycerol) (DPPG), 1,2-dierucoyl-sn-glycero-3-phospho-rac-(1-glycerol) (DEPG), 1,2-dilauroyl-sn-glycero-3-phospho-rac-(1-glycerol) (DLPG), 1,2-dimyristoyl-sn-glycero-3-phospho-rac-(1-glycerol) (DMPG), 1,2-dioleoyl-sn-glycero-3-phospho-rac-(1-glycerol) (DOPG), 1,2-distearoyl-sn-glycero-3-phospho-rac-(1-glycerol) (DSPG), 1-palmitoyl-2-oleoyl-sn-glycero-3-phospho-rac-(1-glycerol) (POPG), or salts thereof, for example, the corresponding sodium salts, ammonium salts, or combinations of the salts thereof. In some such embodiments, the amphipathic lipid comprises phosphatidylcholine, or phosphatidylglycerol or salts thereof, or combinations thereof. In some embodiments, the phosphatidyl choline is DEPC. In some embodiments, the phosphatidyl glycerol is DPPG. According to the invention, the amphipathic lipid comprises DEPC and DPPG. In further embodiments, the DEPC and the DPPG are present in MVLs in a mass ratio of DEPC:DPPG of about 15:1 to about 20:1, or about 17:1. In further embodiments, the total DEPC and DPPG in the MVLs suspension is in a mass ratio of about 7:1 to about 10:1, or about 8:1.

In some embodiments, suitable neutral lipids in the volatile water-immiscible solvent solution may include but are not limited to triglycerides, propylene glycol esters, ethylene glycol esters, and squalene. Non-limiting exemplary triglycerides useful in the instant formulations and processes are triolein (TO), tripalmitolein, trimyristolein, trilinolein, tributyrin, tricaproin, tricaprylin (TC), and tricaprin. The fatty acid chains in the triglycerides useful in the present application can be all the same, or not all the same (mixed chain triglycerides), or all different. In one embodiment, the neutral lipid comprises or is tricaprylin. In further embodiments, the volatile water-immiscible solvent solution in step (a) of the process may further comprise cholesterol and/or a plant sterol.

In some embodiments of the process described herein, the mixing in step (a) is performed using a first mixer at a high shear speed. In some embodiments, the high sheer speed is from about 1100 rpm to about 1200 rpm, for example, 1100 rpm, 1110 rpm, 1120 rpm, 1130 rpm, 1140 rpm, 1150 rpm, 1160 rpm, 1170 rpm, 1180 rpm, 1190 rpm, or 1200 rpm, or a range defined by any of the two preceding values. In some embodiment, the high sheer speed is about 1150 rpm. In some embodiments, the mixing in step (a) is performed for about 65 minutes, 66 minutes, 67 minutes, 68 minutes, 69 minutes, 70 minutes, 71 minutes, 72 minutes, 73 minutes, 74 minutes or 75 minutes. Proper mixing rate is important for forming the first emulsion droplets in a proper size range, which is important to the final product yield, the MVL particle stability and release properties. It was observed that when the mixing speed is too low or too high, the droplets formed in the first emulsion were either too big or too small. In some further embodiments, the first mixer used in step (a) of the process has a blade diameter of between about 8 inch to about 10 inch. In further embodiments, the first mixer used in step (a) of the process is not a static mixer. In further embodiments, the mixing in step (a) is performed at a temperature of about 21 °C to about 23 °C.

In some embodiments of the process described herein, the mixing in step (b) is performed using a second mixer at a low shear speed. In some embodiments, the low sheer speed is from about 450 rpm to about 510 rpm, for example, 450 rpm, 455 rpm, 460 rpm, 465 rpm, 470 rpm, 475 rpm, 480 rpm, 485 rpm, 490 rpm, 495 rpm, 500 rpm, 505 rpm, or 510 rpm, or a range defined by any of the two preceding values. In some embodiment, the low sheer speed is about 495 rpm. In some embodiments, the mixing in step (b) is performed for about 60 seconds, 61 seconds, 62 seconds, 63 seconds, 64 seconds, or 65 seconds. In some further embodiments, the second mixer used in step (b) of the process has a blade diameter of between about 10 inch to about 15 inch, for example, 10 inch, 11 inch, 12 inch, 13 inch, or 14 inch. It is noted that 1 inch converts to 2,54 centimetres. In further embodiments, the second mixer used in step (b) of the process is not a static mixer. In further embodiments, the mixing in step (b) is performed at a temperature of about 21 °C to about 23 °C. The water-in-oil-in water (w/o/w) second emulsion is not as stable as the first emulsion. As such, a low shear speed was used in mixing step to reduce the disruption of the spherules formed in this step. In addition, the mixing time in step (b) is also important to yield the final MVL particles in the target diameters and have the desired release properties. If mixing time is too short, it led to a larger particle size.

In some embodiments of the process described herein, the second aqueous solution comprises one or more pH modifying agents. The pH modifying agents that may be used in the present MVL formulations are selected from organic acids, organic bases, inorganic acids, or inorganic bases, or combinations thereof. Suitable organic bases that can be used in the present application include, but are not limited to histidine, arginine, lysine, tromethamine (Tris), etc. Suitable inorganic bases that can be used in the present application include, but are not limited to sodium hydroxide, calcium hydroxide, magnesium hydroxide, potassium hydroxide, etc. Suitable inorganic acids (also known as mineral acids) that can be used in the present application include, but are not limited to hydrochloric acid (HCl), sulfuric acid (H₂SO₄), phosphoric acid (H₃PO₄), nitric acid (HNO₃), etc. Suitable organic acids that can be used in the present application include, but are not limited to acetic acid, aspartic acid, citric acid, formic acid, glutamic acid, glucuronic acid, lactic acid, malic acid, tartaric acid, etc. In one embodiment, the pH modifying agent comprises lysine.

In some embodiments of the process described herein, the second aqueous solution comprises one or more tonicity agents. Tonicity agents sometimes are also called osmotic agents. Non-limiting exemplary osmotic agents suitable for the MVL formulation of the present application include monosaccharides (e.g., glucose, and the like), disaccharides (e.g., sucrose and the like), polysaccharide or polyols (e.g., sorbitol, mannitol, Dextran, and the like), or amino acids. In some embodiments, one or more tonicity agents may be selected from an amino acid, a sugar, or combinations thereof. In some further embodiments, one or more tonicity agents are selected from dextrose, sorbitol, sucrose, lysine, or combinations thereof. In one embodiment, the tonicity agent comprises dextrose. According to the invention, the second aqueous solution comprises lysine and dextrose.

In some embodiments of the process described herein, the volatile water-immiscible organic solvent comprises or is methylene chloride (CH₂Cl₂). The organic solvent is substantially removed by exposing the second emulsion to a gas atmosphere. Organic solvent may be removed by blowing a gas over the second emulsion, or sparging gas in the second emulsion, or spraying the second emulsion into a chamber with a continuous stream of circulating gas.

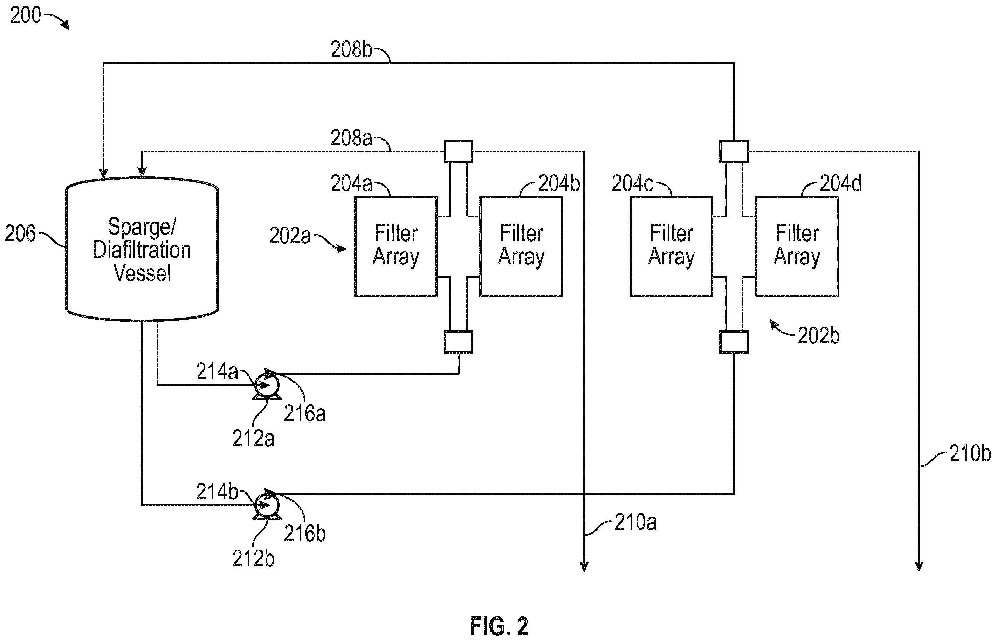

In some embodiments of the process described herein, wherein step (e) is performed using two sets of filtration modules, wherein each set of the filtration modules operate independently of the other. In further embodiments, each set of the filtration module comprises five or more hollow fiber filters, each having a membrane pore size from about 0.1 µm to about 0.2 µm. One embodiment of the filtration modules are illustrated in FIG. 2.

In some embodiments of process described herein, the diafiltration step (e) is performed multiple times until the aqueous supernatant of the second aqueous suspension is substantially replaced with the saline solution.

In some embodiments of process described herein, step (f) may be performed multiple times until a target concentration of bupivacaine MVLs is reached. In some further embodiments, the final aqueous suspension of bupivacaine encapsulated multivesicular liposomes is transferred to a bulk product vessel.

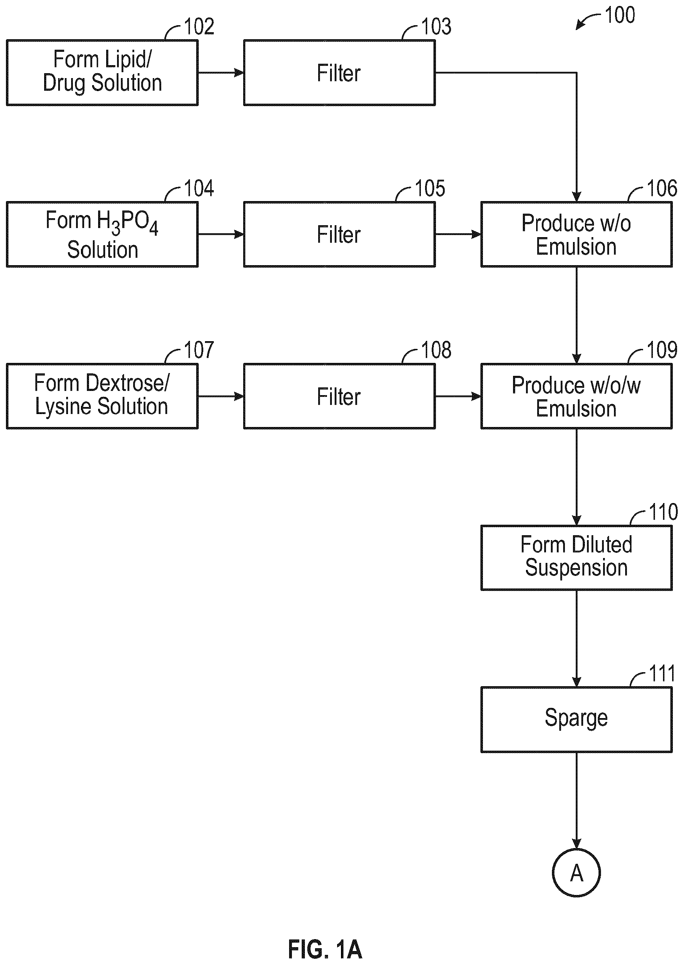

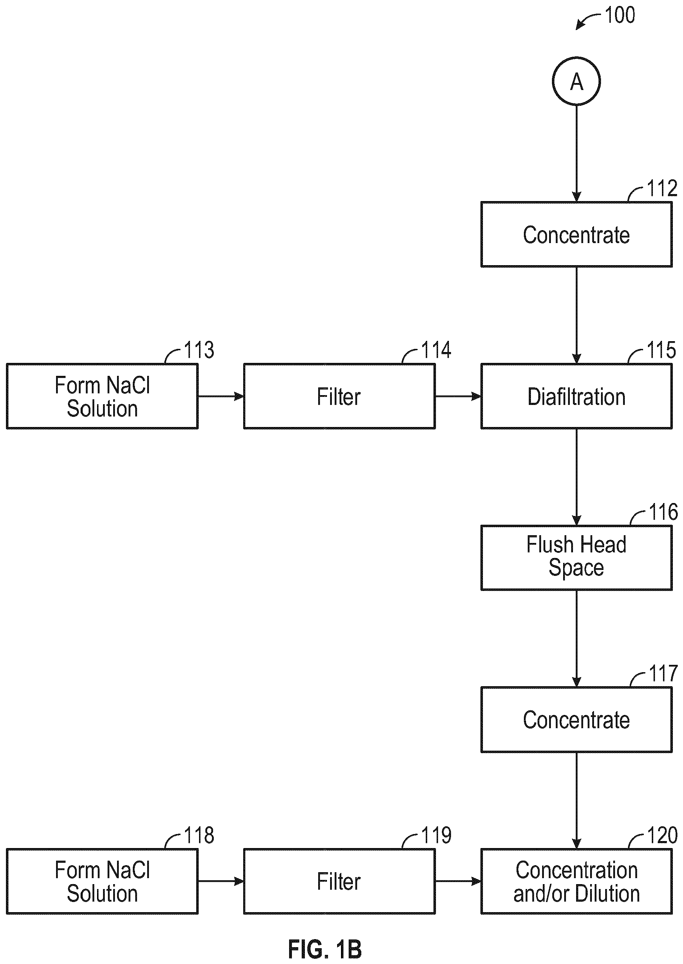

FIGs. 1A-1B are process flow charts, each depicting a portion of the bupivacaine MVLs manufacturing process 100 according to some embodiments described herein. The circled A symbol indicates the connection point between FIG. 1A and FIG. 1B. As shown in FIGS. 1A-1B, bupivacaine MVLs is produced via an aseptic double-emulsion process. The bulk manufacturing system is a closed, sterilized system into which all process solutions are sterile-filtered through 0.2 µm filters.

As shown in FIG. 1A, the process 100 includes a step 102, wherein DEPC, DPPG, cholesterol, tricaprylin, and bupivacaine are dissolved in methylene chloride to form a lipid/drug solution 102. At a step 103, the lipid solution is filtered through a 0.2 µm membrane filter into a sterilized vessel. At a step 104, phosphoric acid is dissolved in WFI (water for injection) to form a H₃PO₄ solution. At a step 105, the H₃PO₄ solution is filtered through a 0.2 µm membrane filter into a sterilized vessel. Under aseptic conditions, the filtered lipid/drug solution is combined with the filtered H₃PO₄ solution in a volume ratio of 1:1 at an emulsification step 106 using agitation to produce a w/o emulsion (i.e., first emulsion). High shear mixing of the lipid/drug solution with the phosphoric acid solution is performed, wherein bupivacaine is ionized by the phosphoric acid and partitions into the internal aqueous phase. At a step 107, lysine and dextrose are combined in WFI to form a dextrose/lysine solution. At a step 108, the dextrose/lysine solution is filtered through a 0.2 µm membrane filter into a sterilized vessel. Under aseptic conditions, the filtered dextrose/lysine solution is added to the w/o emulsion in a volume ratio of approximately 2.5:1 at an emulsification step 109 using agitation to produce a w/o/w emulsion (i.e., second emulsion). At emulsification step 109, agitation is performed at lower shear, producing a water-in-oil-in-water (w/o/w) emulsion with the majority of the bupivacaine resident in the internal aqueous phase. Additional filtered dextrose/lysine solution is added to the w/o/w emulsion at a dilution step 110 to form a diluted suspension of MVLs and bringing the final volume ratio to approximately 20:1 (dextrose/lysine solution to water-in-oil emulsion) with mixing. At a step 111, the diluted suspension of MVLs is sparged with sterile nitrogen to remove the majority of the methylene chloride.

FIG. 1B depicts additional steps of the process 100. After sparging at step 111, the diluted suspension of bupivacaine MVLs is concentrated via aseptic microfiltration at a step 112 to a bupivacaine concentration of approximately 4.5 mg/mL.

At a step 113, a NaCl solution is formed by dissolving sodium chloride in WFI. At a step 114, the NaCl solution (i.e., saline solution) is filtered through a 0.2 µm membrane filter. Under aseptic conditions, the bupivacaine MVLs concentrate formed at step 112 is subjected to crossflow filtration by at least four volumes of the filtered NaCl solution through introduction of the filtered NaCl solution into a crossflow filtration apparatus or system through multiple 0.2 µm hollow fiber filter membrane unit filters at a diafiltration step 115. Diafiltration step 115 is used to remove unencapsulated bupivacaine, lysine, dextrose and residual methylene chloride, thereby reducing the suspension volume and increasing the concentration of the bupivacaine MVLs in the suspension. At a step 116, sterile nitrogen is used to flush the headspace of the crossflow filtration apparatus or system to further reduce residual methylene chloride content. The solution is further concentrated via aseptic microfiltration in concentrate step 117 to form an initial bulk suspension of MVLs at a target weight/volume that corresponds to a bupivacaine concentration of 11.3-16.6 mg/mL. The bulk product is then transferred into a sterilized holding vessel. The initial bulk suspension of MVLs is sampled and bupivacaine concentration is measured. Optionally, if the initial bulk suspension of MVLs is designated to be filled as an individual lot, the initial bulk suspension of MVLs is concentrated further via sedimentation (gravitational settling) and/or decantation to a bupivacaine concentration of approximately 13.3 mg/mL, or alternatively diluted with a filtered NaCl solution to a bupivacaine concentration of approximately 13.3 mg/mL at a decantation and/or dilution step 120 to form an adjusted bulk suspension of MVLs. The saline solution that is optionally used at step 120 can be formed by dissolving sodium chloride in WFI at a step 118 and filtered through a 0.2 µm membrane filter at a step 119.

### Tangential Flow Filtration Modules

Some embodiments of the present application relates to a crossflow filtration system comprising: a diafiltration vessel; and a plurality of independently operating crossflow modules, each crossflow module of the plurality of independently operating crossflow modules comprising at least one filter array, each filter array comprising a plurality of hollow fiber filters, wherein each crossflow module of the plurality of independently operating crossflow modules is connected to a retentate conduit, a permeate conduit, and a rotary lobe pump. In some embodiments, the crossflow filtration system may be used in the microfiltration and/or diafiltration step of the commercial process described herein.

In some embodiments, each crossflow module comprises two filter arrays. In some embodiments, each crossflow module comprises at least five hollow fiber filters. In some such embodiments, each filter array comprises at least two hollow fiber filters.

In some embodiments, the plurality of independently operating crossflow modules comprises a first crossflow module and a second crossflow module, wherein the first crossflow module is coupled to a first rotary lobe pump and the second crossflow module is coupled to a second rotary lobe pump operating independently of the first rotary lobe pump. In some further embodiments, the first crossflow module is coupled to the diafiltration vessel by a first retentate conduit to facilitate flow of retentate from the first crossflow module to the diafiltration vessel, and wherein the second crossflow module is coupled to the diafiltration vessel by a second retentate conduit to facilitate flow of retentate from the second crossflow module to the diafiltration vessel. In some further embodiments, the first rotary lobe pump comprises a fluid outlet coupling the first rotary lobe pump to the first crossflow module, and wherein the second rotary lobe pump comprises a fluid outlet coupling the second rotary lobe pump to the first crossflow module. In some further embodiments, the first rotary lobe pump comprises a fluid inlet coupling the first rotary lobe pump to the diafiltration vessel, and wherein the second rotary lobe pump comprises a fluid inlet coupling the second rotary lobe pump to the diafiltration vessel.

In some embodiments, the first crossflow module operates independently from the second crossflow module. In some such embodiments, only one of the first crossflow module and the second crossflow module is in use during the operation of the crossflow filtration system. In other embodiments, both the first crossflow module and the second crossflow module are in use during the operation of the crossflow filtration system.

In some embodiments, each of the plurality of independently operating crossflow modules comprises a microfiltration mode and a diafiltration mode.

In some embodiments, the crossflow filtration system further comprises a nitrogen sparging module to blow a stream nitrogen over the retentate in the diafiltration vessel.

Some further embodiments of the present application relate to a process of manufacturing bupivacaine encapsulated multivesicular liposomes using the cross-flow module described herein, the process comprising:
reducing a first aqueous suspension of bupivacaine encapsulated MVLs having a first volume by microfiltration to provide a second aqueous suspension of bupivacaine encapsulated MVLs having a second volume;
exchanging the aqueous supernatant of the second aqueous suspension with a saline solution by diafiltration to provide a third aqueous suspension of bupivacaine encapsulated MVLs having a third volume; and
further reducing the third volume of the third aqueous suspension by microfiltration to provide a final aqueous suspension of bupivacaine encapsulated MVLs having a target concentration of bupivacaine.

In some embodiments, the process further comprises blowing a stream of nitrogen over the second aqueous suspension during the diafiltration/saline exchange step. In some further embodiments, the diafiltration include at least two, three, four or five exchange volumes of the saline solution such that the aqueous supernatant of the second aqueous suspension is substantially (e.g., at least 95%, 96%, 97%, 98%, 99%) replaced by the saline solution.

Some further embodiments relate to a composition of bupivacaine encapsulated multivesicular liposomes prepared by the process utilizing the crossflow filtration system described herein.

FIG. 2 depicts an embodiment of a crossflow filtration system 200 for use in a diafiltration step of a commercial scale manufacturing process as described herein, such as step 115 of the process 100. The system 200 includes independently operating crossflow modules 202a and 202b. Crossflow module 202a includes a filter array 204a and a filter array 204b. Crossflow module 202b includes a filter array 204c and a filter array 204d. Each filter array 204a-d may include two or more hollow fiber filters. In some embodiments, each filter array includes five or more hollow fiber filters.

The system may be connected to a sparge/diafiltration vessel 206. Retentate can flow from the crossflow module 202a to the vessel 206 via a retentate return conduit 208a. Retentate can flow from the crossflow module 202b to the vessel 206 via a retentate return conduit 208b. Permeate can flow from the crossflow module 202a for removal from the system 200 via a permeate conduit 210a. Permeate can flow from the crossflow module 202b for removal from the system 200 via a permeate conduit 210b.

The system 200 may include or be used in conjunction with two independently operating rotary lobe pumps 212a and 212b. The pump 212a includes a fluid inlet 214a and a fluid outlet 216a. The pump 212b includes a fluid inlet 214b and a fluid outlet 216b. The pump 212a is connected to the vessel 206 via the inlet 214a and connected to crossflow module 202a via the outlet 216a. The pump 212b is connected to the vessel 206 via the inlet 214b and connected to the crossflow module 202b via the outlet 216b.

In some embodiments of the process described herein, the crossflow filtration system utilizes two independent rotary lobe pumps providing retentate flow to independent arrays of five hollow fiber filter housings. This configuration allows for smaller pipe diameters to allow for turbulent flow. In addition, the filtration module design allows for two filter arrays to be in-use during bulk operation while two filter arrays are being cleaned and sterilized in preparation for the next bulk production run. This configuration allows for shorter cycle times and increased manufacturing capacity. Furthermore, the improved filtration module design allows for independent hollow fiber filter housing isolation. This functionality automatically detects and isolates individual filter integrity failures, allowing the bulk cycle to proceed without offline testing and recleaning. In some further embodiments, the process may further comprise an additional product recovery step from one of the two filter array and/or an saline flush step, to allow for nearly complete product recover from the transfer lines and thereby increasing product yield.

In some embodiments of the process described herein, the final aqueous suspension of bupivacaine encapsulated multivesicular liposomes has a volume of about 150 L to about 250 L. In one embodiment, the final aqueous suspension of bupivacaine encapsulated multivesicular liposomes has a volume of about 200 L. In another embodiment, the final aqueous suspension of bupivacaine encapsulated multivesicular liposomes has a volume of about 225 L. In some embodiments, the percent packed particle volume (% PPV) of the final aqueous suspension of bupivacaine encapsulated multivesicular liposomes is about 32% to about 44%, about 35% to about 40%, or about 36% to about 38%. In some such embodiments, the target concentration of the bupivacaine in the final aqueous suspension (i.e., bulk product suspension) is from about 11.3 mg/mL to about 17 mg/mL, or about 12.6 mg/mL to about 17 mg/mL. In further embodiments, the final product target concentration of the bupivacaine in the aqueous suspension is about 13.3 mg/mL. In some embodiments, the final aqueous suspension of bupivacaine MVLs comprises less than 5%, 4%, 3%, 2% or 1% unencapsulated bupivacaine, wherein the amount of unencapsulated bupivacaine is calculate based on the total weight of the bupivacaine in the aqueous suspension. In some embodiments, the d₅₀ of the multivesicular liposomes in the final aqueous suspension is about 24 µm to about 31 µm. In one embodiments, the d₅₀ of the multivesicular liposomes in the final aqueous suspension is about 27 µm. In some embodiments, the internal pH of the bupivacaine encapsulated multivesicular liposomes is about 5.5. In some such embodiments, the lysine concentration inside the bupivacaine multivesicular liposome particles (i.e., internal lysine concentration or encapsulated lysine concentration) is about 0.08 mg/mL.. In further embodiments, the internal lysine concentration is about 0.03 mg/mL, where the lysine concentration is measured when the MVL particles are in the aqueous suspension. In some embodiments, the external pH of the bupivacaine encapsulated multivesicular liposomes is about 7.0 to about 7.4. As used herein, "internal pH" of the bupivacaine MVLs refer to the pH of the internal aqueous chambers of the MVL particles. The pH of the aqueous suspension of the bupivacaine MVLs is also referred to as the "external pH" of the bupivacaine MVLs. In some embodiments, the external pH of the bupivacaine MVLs are measured during the product's shelf life under the storage condition between 2-8 °C. When the bupivacaine MVLs are stored at ambient temperature at extended period of time, the external pH of the composition may drop below the 7.0-7.4 range partially due to the accelerated lipid hydrolysis.

### Bupivacaine Multivesicular Liposomes Prepared by the New Process

MVLs are a group of unique forms of synthetic membrane vesicles that are different from other lipid-based delivery systems such as unilamellar liposomes and multilamellar liposomes (Bangham, et al., J Mol. Bio., 13:238-252, 1965). The main structural difference between multivesicular liposomes and unilamellar liposomes (also known as unilamellar vesicles, "ULVs"), is that multivesicular liposomes contain multiple aqueous chambers per particle. The main structural difference between multivesicular liposomes and multilamellar liposomes (also known as multilamellar vesicles, "MLVs"), is that in multivesicular liposomes the multiple aqueous chambers are non-concentric. Multivesicular liposomes generally have between 100 to 1 million chambers per particle and all the internal chambers are interconnected by shared lipid-bilayer walls that separate the chambers. The presence of internal membranes distributed as a network throughout multivesicular liposomes may serve to confer increased mechanical strength to the vesicle. The particles themselves can occupy a very large proportion of the total formulation volume. Such formulation is intended to prolong the local delivery of bupivacaine, thereby enhancing the duration of action of the reduction of pain.

The bupivacaine MVLs produced by the process described herein have improved stability over the commercial Exparel^{®} product. It was observed that the bupivacaine MVL particles produced by the process described herein have lower lipid hydrolysis byproducts compared to the commercial Exparel^{®} product under the same incubation condition. In addition, the bupivacaine MVL particles produced by the process described herein has higher internal lysine and dextrose concentrations and more desirable internal pH, which may improve MVL particle strength during product transportation, as well as lipid membrane stability.

Some embodiments of the present disclosure relate to a composition of bupivacaine encapsulated multivesicular liposomes (MVLs) prepared by a commercial scale process described herein, the commercial scale process comprising:
(a) mixing a first aqueous solution comprising phosphoric acid with a volatile water-immiscible solvent solution to form a water-in-oil first emulsion, wherein the volatile water-immiscible solvent solution comprises bupivacaine, 1, 2-dierucoylphosphatidylcholine (DEPC), 1, 2-dipalmitoyl-sn-glycero-3 phospho-rac-(1-glycerol) (DPPG), and at least one neutral lipid;
(b) mixing the water-in-oil first emulsion with a second aqueous solution to form a water-in-oil-in-water second emulsion;
(c) removing the volatile water-immiscible solvent from the water-in-oil-in-water second emulsion to form a first aqueous suspension of bupivacaine encapsulated MVLs having a first volume;
(d) reducing the first volume of the first aqueous suspension of bupivacaine encapsulated MVLs by microfiltration to provide a second aqueous suspension of bupivacaine encapsulated MVLs having a second volume;
(e) exchanging the aqueous supernatant of the second aqueous suspension with a saline solution by diafiltration to provide a third aqueous suspension of bupivacaine encapsulated MVLs having a third volume; and
(f) further reducing the third volume of the third aqueous suspension by microfiltration to provide a final aqueous suspension of bupivacaine encapsulated MVLs having a target concentration of bupivacaine;

wherein all steps are carried out under aseptic conditions; and
wherein the erucic acid concentration in the composition is 23 µg/mL or less after the composition is stored at 25°C for one month. In one embodiment, the erucic acid concentration in the composition is about 22.7 µg/mL after the composition is stored at 25°C for one month.

In some embodiments, the final aqueous suspension of bupivacaine encapsulated MVLs described in the process is the composition of the bupivacaine MVLs described herein. In other embodiments, the concentration of the final aqueous suspension of bupivacaine encapsulated MVLs described in the process may be further adjusted with a saline solution to provide the composition of the bupivacaine MVLs described herein. In some embodiments, the composition has a pH of about 7.1 after the composition is stored at 25°C for one month.

In some further embodiments, the erucic acid concentration in the composition is about 38 µg/mL or less after the composition is stored at 25°C for two months. In one embodiment, the erucic acid concentration in the composition is about 37.3 µg/mL after the composition is stored at 25°C for two months. In some such embodiments, the composition has a pH of about 7.1 after the composition is stored at 25°C for two months.

In some further embodiments, the erucic acid concentration in the composition is about 54 µg/mL or less after the composition is stored at 25°C for three months. In one embodiment, the erucic acid concentration in the composition is about 53 µg/mL after the composition is stored at 25°C for three month. In some such embodiments, the composition has a pH of about 6.9 after the composition is stored at 25°C for three months.

In some further embodiments, the erucic acid concentration in the composition is about 100 µg/mL or less after the composition is stored at 25°C for six months. In one embodiment, the erucic acid concentration in the composition is about 98.7µg/mL after the composition is stored at 25°C for six months. In further embodiments, compositions or batches of bupivacaine MVLs produced by the process described herein consistently contain less than 109 µg/mL erucic acid, for example, about 99 µg/mL or less erucic acid after the compositions or batches are stored at 25°C for six months. In some further embodiments, the composition has a pH of about 6.5 after the composition is stored at 25°C for six months. In some further embodiments, the batches have a pH of about 6.5 after the batches are stored at 25°C for six months.

In some embodiments, the composition of bupivacaine MVLs comprises the following lipid components: DEPC, DPPG, cholesterol and tricaprylin. In some embodiments, the total concentrations of the lipid components in the composition are the following: DEPC (about 7.0 mg/mL), DPPG (about 0.9 mg/mL), cholesterol (about 4.2 mg/mL), tricaprylin (about 1.6 mg/mL). Since DEPC has the highest concentration of all the lipid components, the hydrolysis byproducts of DEPC is used as the marker to assess lipid stability of the MVLs. The hydrolysis byproducts of DEPC include erucic acid and lyso-DEPC (1- and 2-isomers). Lyso-DEPC is formed by hydrolysis of DEPC. Lyso-DEPC can further hydrolyze to glycerophosphocholine and erucic acid. In some embodiments, the erucic acid concentration in the composition of bupivacaine MVLs produced by the process described herein is at least about 1%, 2%, 3%, 4%, 5%, 6%, 7%, 8%, 9%, 10%, 11%, 12%, 13%, 14% or 15% less than the erucic acid concentration in the Exparel^{®} product manufactured by the current commercial process, under the same incubation condition. In some such embodiments, the incubation condition is at 25° for 1 month, 2 months, 3 months, or 6 months.

Some additional embodiments of the present disclosure relate to a composition of bupivacaine encapsulated multivesicular liposomes (MVLs) prepared by a commercial scale process described herein, the commercial scale process comprising:
(a) mixing a first aqueous solution comprising phosphoric acid with a volatile water-immiscible solvent solution to form a water-in-oil first emulsion, wherein the volatile water-immiscible solvent solution comprises bupivacaine, 1, 2-dierucoylphosphatidylcholine (DEPC), 1, 2-dipalmitoyl-sn-glycero-3 phospho-rac-(1-glycerol) (DPPG), and at least one neutral lipid;
(b) mixing the water-in-oil first emulsion with a second aqueous solution to form a water-in-oil-in-water second emulsion, wherein the second aqueous solution comprises lysine and dextrose;
(c) removing the volatile water-immiscible solvent from the water-in-oil-in-water second emulsion to form a first aqueous suspension of bupivacaine encapsulated MVLs having a first volume;
(d) reducing the first volume of the first aqueous suspension of bupivacaine encapsulated MVLs by microfiltration to provide a second aqueous suspension of bupivacaine encapsulated MVLs having a second volume;
(e) exchanging the aqueous supernatant of the second aqueous suspension with a saline solution by diafiltration to provide a third aqueous suspension of bupivacaine encapsulated MVLs having a third volume; and
(f) further reducing the third volume of the third aqueous suspension by microfiltration to provide a final aqueous suspension of bupivacaine encapsulated MVLs having a target concentration of bupivacaine;

wherein all steps are carried out under aseptic conditions; and
wherein the internal pH of the bupivacaine encapsulated MVLs in the composition is about 5.49 to about 5.51, or about 5.50. In some embodiments, the internal pH is measured after the composition has been stored at about 2-8 °C for at least 3 months, 6 months or 9 months. In one embodiment, the internal pH is measured after the composition has been stored at about 2-8 °C for about 9 months.

In some embodiments, the final aqueous suspension of bupivacaine encapsulated MVLs described in the process is the composition of the bupivacaine MVLs described herein. In other embodiments, the concentration of the final aqueous suspension of bupivacaine encapsulated MVLs described in the process may be further adjusted with a saline solution to provide the composition of the bupivacaine MVLs described herein.

In some embodiments of the composition described herein, the lysine concentration inside the bupivacaine encapsulated MVL particles of the composition (internal lysine concentration or encapsulated lysine concentration) is about 0.030 mg/mL to about 0.032 mg/mL. In some such embodiment, the internal lysine concentration is measured when the bupivacaine MVLs are suspended in an aqueous suspension with %PPV about 36.5%. In some further embodiments, the internal lysine concentration inside the bupivacaine encapsulated MVL particles is about 0.031 mg/mL.

In some embodiments of the composition described herein, the dextrose concentration inside the bupivacaine encapsulated MVL particles of the composition (internal dextrose concentration or encapsulated dextrose concentration) is about 1.25 mg/mL to about 1.32 mg/mL. In some such embodiment, the internal dextrose concentration is measured when the bupivacaine MVLs are suspended in an aqueous suspension with %PPV about 36.5%. In some further embodiments, the internal dextrose concentration inside the bupivacaine encapsulated MVL particles is about 1.29 mg/mL.

Although it is expected that only phosphoric acid reside inside the internal aqueous chambers of the MVL particles (i.e., the first emulsion is formed by mixing the phosphoric acid aqueous solution with a volatile water-immiscible solvent solution). However, there are also very small amounts of lysine and dextrose encapsulated inside the internal aqueous chambers during the formation of the second emulsion, which ultimately forms the MVL particles. In some embodiments, the lysine concentration in the bupivacaine MVLs produced by the process described herein is at least about 5%, 10%, 15%, 20%, 25%, or 30% more than the encapsulated lysine concentration in the Exparel^{®} product manufactured by the current commercial process. Since lysine is also a pH modifying agent, the small change in lysine concentration also results in the increase of the internal pH of the bupivacaine MVL particles of about 5.49 to about 5.51, or about 5.50, as compared to the internal pH of about 5.34 in a batch of Exparel^{®} product manufactured by the current commercial process. In some such embodiments, the increase of the internal pH is characterized by the decrease in [H⁺] concentration (i.e., pH = -log [H⁺]). In some embodiments, the decrease in [H⁺] concentration in the internal aqueous chambers of the bupivacaine MVLs is at least about 5%, 10%, 15%, 20%, 25% or 30%. As reported by Grit M. *et al.,* phospholipids such as phosphatidylcholine have the lowest rate of hydrolysis regardless of the temperature of their storage at pH 6.5. *See* J. Pharm. Sci. 1993;82(4):362-366. As such, the closer the internal pH is to 6.5, the lower the lipid hydrolysis. In some further embodiments, the dextrose concentration in the bupivacaine MVLs produced by the process described herein is at least about 2%, 5%, 7.5%, 10%, 12.5%, 15%, 17.5% or 20% more than the encapsulated dextrose concentration in the Exparel^{®} product manufactured by the current commercial process.

In some embodiments of the composition described herein, the mixing in step (a) is performed using a first mixer at a high shear speed. In some embodiments, the high sheer speed is from about 1100 rpm to about 1200 rpm, for example, 1100 rpm, 1120 rpm, 1130 rpm, 1140 rpm, 1150 rpm, 1160 rpm, 1170 rpm, 1180 rpm, 1190 rpm, or 1200 rpm, or a range defined by any two of the preceding values. In some embodiment, the high sheer speed is about 1150 rpm. In some embodiments, the mixing in step (a) is performed for about 65 minutes, 66 minutes, 67 minutes, 68 minutes, 69 minutes, 70 minutes, 71 minutes, 72 minutes, 73 minutes, 74 minutes or 75 minutes. In some further embodiments, the first mixer used in step (a) of the process is a mixer having a blade diameter of between about 8 inch to about 10 inch. In further embodiments, the first mixer used in step (a) of the process is not a static mixer. In further embodiments, the mixing in step (a) is performed at a temperature of about 21 °C to about 23 °C.

In some embodiments of the composition described herein, the mixing in step (b) is performed using a second mixer at a low shear speed. In some embodiments, the low sheer speed is from about 450 rpm to about 510 rpm, for example, 450 rpm, 455 rpm, 460 rpm, 465 rpm, 470 rpm, 475 rpm, 480 rpm, 485 rpm, 490 rpm, 495 rpm, 500 rpm, 505 rpm, or 510 rpm, or a range defined by any of the two preceding values. In some embodiment, the low sheer speed is about 495 rpm. In some embodiments, the mixing in step (b) is performed for about 60 seconds, 61 seconds, 62 seconds, 63 seconds, 64 seconds, or 65 seconds. In some further embodiments, the second mixer used in step (b) of the process has a blade diameter of between about 10 inch to about 15 inch, for example, 10 inch, 11 inch, 12 inch, 13 inch, or 14 inch. In further embodiments, the second mixer used in step (b) of the process is not a static mixer. In further embodiments, the mixing in step (b) is performed at a temperature of about 21 °C to about 23 °C.

In some embodiments of the composition described herein, the composition of bupivacaine encapsulated multivesicular liposomes may have a final volume of about 150 L to about 250 L, or about 200 L to about 250 L, before being filled into individual containers for human administration. In other embodiments, the composition of bupivacaine encapsulated MVLs may have a volume of 10 mL or 20 mL for a single dose administration. In some embodiments, the percent packed particle volume (% PPV) of the composition of bupivacaine encapsulated MVLs is about 32%, 33%, 34%, 35%, 36%, 37%, 38%, 39%, 40%, 41%, 42%, 43% or 44%. In some such embodiments, the concentration of the bupivacaine in the composition is from about 11.3 mg/mL to about 17 mg/mL, or about 12.6 mg/mL to about 17 mg/mL. In one embodiment, the concentration of the bupivacaine in the composition is about 13.3 mg/mL. In further embodiments, the single dose administration provides up to 266 mg bupivacaine. In further embodiments, the composition comprises less than 5%, 4%, 3%, 2% or 1% unencapsulated bupivacaine, wherein the amount of unencapsulated bupivacaine is calculated based on the total weight of the bupivacaine in the composition. In some embodiments, the d₅₀ of the multivesicular liposomes in the composition is about 24 µm to about 31 µm. In one embodiments, the d₅₀ of the multivesicular liposomes in the composition is about 27 µm.

### Bupivacaine Multivesicular Liposomes

Some aspect of the present disclosure relates to a composition of bupivacaine encapsulated multivesicular liposomes (MVLs), comprising: bupivacaine residing inside a plurality of internal aqueous chambers of the MVLs separated by lipid membranes, wherein the lipid membranes comprise 1, 2-dierucoylphosphatidylcholine (DEPC), 1,2-dipalmitoyl-sn-glycero-3 phospho-rac-(1-glycerol) (DPPG), and at least one neutral lipid; and an aqueous medium in which the bupivacaine encapsulated MVLs are suspended; wherein erucic acid concentration in the composition is 23 µg/mL or less (e.g., about 22.7 µg/mL) after the composition is stored at 25°C for one month. In some embodiments, the composition has an initial pH of about 7.4. In some further embodiments, the erucic acid concentration in the composition is about 38 µg/mL or less (e.g., about 37.3 µg/mL) after the composition is stored at 25°C for two months. In some such embodiments, the composition has a pH of about 7.1 after the composition is stored at 25°C for two months. In some further embodiments, the erucic acid concentration in the composition is about 54 µg/mL or less (e.g., about 53.0 µg/mL) after the composition is stored at 25°C for three month. In some such embodiments, the composition has a pH of about 6.9 after the composition is stored at 25°C for three months. In some further embodiments, the erucic acid concentration in the composition is about 100 µg/mL or less (e.g., about 98.7 µg/mL) after the composition is stored at 25°C for six months. In some further embodiments, the composition has a pH of about 6.5 after the composition is stored at 25°C for six months. In some further embodiments, the lipid membranes further comprise cholesterol and tricaprylin. In further embodiments, the compositions of bupivacaine MVLs described herein relates to batches of bupivacaine MVLs and consistently comprise less than 109 µg/mL erucic acid, for example about 99 µg/mL or less erucic acid after the batches are stored at 25°C for six months.

In some embodiments, the erucic acid concentration in the composition of bupivacaine MVLs is at least about 1%, 2%, 3%, 4%, 5%, 6%, 7%, 8%, 9%, 10%, 11%, 12%, 13%, 14% or 15% less than the erucic acid concentration in the Exparel^{®} product currently on the market, under the same incubation conditions. In some such embodiments, the incubation condition is at 25° for 1 month, 2 months, 3 months, or 6 months.

Some additional aspect of the present disclosure relates to a composition of bupivacaine encapsulated multivesicular liposomes (MVLs), comprising: bupivacaine residing inside a plurality of internal aqueous chambers of the MVLs separated by lipid membranes, wherein the lipid membranes comprise 1, 2-dierucoylphosphatidylcholine (DEPC), 1, 2-dipalmitoyl-sn-glycero-3 phospho-rac-(1-glycerol) (DPPG), and at least one neutral lipid; and an aqueous medium in which the bupivacaine encapsulated MVLs are suspended; wherein the internal pH of the bupivacaine encapsulated MVLs is about 5.49 to about 5.51, or about 5.50. In some embodiments of the composition described herein, the internal lysine concentration of the bupivacaine encapsulated MVLs composition is about 0.030 mg/mL to about 0.032 mg/mL. In some further embodiments, the internal lysine concentration of the bupivacaine encapsulated MVLs composition is about 0.031 mg/mL. In some embodiments of the composition described herein, the internal dextrose concentration of the bupivacaine encapsulated MVLs composition is about 1.25 mg/mL to about 1.32 mg/mL. In some further embodiments, the internal dextrose concentration of the bupivacaine encapsulated MVLs composition is about 1.29 mg/mL. In some further embodiments, the lipid membranes further comprise cholesterol and tricaprylin. In some further embodiments, the internal lysine or dextrose concentration are measured when the bupivacaine MVLs are in an aqueous suspension having %PPV from about 36% to about 38% (e.g., about 36.5%).

In some embodiments of the composition described herein, the internal lysine concentration in the bupivacaine MVLs is at least about 5%, 10%, 15%, 20%, 25%, or 30% more than the encapsulated lysine concentration in the Exparel^{®} product currently on the market. In some such embodiments, the small change in lysine concentration also results in the increase of the internal pH of the bupivacaine MVL particles of about 5.49 to about 5.51, or about 5.50, as compared to the internal pH of about 5.34 in the Exparel^{®} product currently on the market. In some such embodiments, the increase of the internal pH is characterized by the decrease in [H⁺] concentration (i.e., pH = -log [H⁺]). In some embodiments, the decrease in [H⁺] concentration in the internal aqueous chambers of the bupivacaine MVLs is at least about 5%, 10%, 15%, 20%, 25% or 30%. In some further embodiments, the internal dextrose concentration in the bupivacaine MVLs is at least about 2%, 5%, 7.5%, 10%, 12.5%, 15%, 17.5% or 20% more than the encapsulated dextrose concentration in the Exparel^{®} currently on the market.

In some further embodiments, the composition of bupivacaine encapsulated MVLs may have a volume of 10 mL or 20 mL for a single dose administration. In some embodiments, the percent packed particle volume (% PPV) of the composition of bupivacaine encapsulated MVLs is about 32%, 33%, 34%, 35%, 36%, 37%, 38%, 39%, 40%, 41%, 42%, 43% or 44%. In some such embodiments, the concentration of the bupivacaine in the composition is from about 11.3 mg/mL to about 17 mg/mL, or about 12.6 mg/mL to about 17 mg/mL. In one embodiment, the concentration of the bupivacaine in the composition is about 13.3 mg/mL. In further embodiments, the composition comprises less than 5%, 4%, 3%, 2% or 1% unencapsulated bupivacaine, wherein the amount of unencapsulated bupivacaine is calculate based on the total weight of the bupivacaine in the composition. In some embodiments, the d₅₀ of the multivesicular liposomes in the composition is about 24 µm to about 31 µm. In one embodiments, the d₅₀ of the multivesicular liposomes in the composition is about 27 µm.

### Methods of Administration

Some embodiments of the present application are related to compositions for use in methods for treating, ameliorating pain comprising administering a pharmaceutical composition comprising bupivacaine MVLs, as described herein, to a subject in need thereof. In some further embodiments, the pain is post surgical pain.

In some embodiments of the methods described herein, the administration is parenteral. In some further embodiments, the parenteral administration may be selected from the group consisting of subcutaneous injection, tissue injection, intramuscular injection, intraarticular, spinal injection, intraocular injection, epidural injection, intrathecal injection, intraotic injection, perineural injection, and combinations thereof. In particular embodiments, the parenteral administration is subcutaneous injection or tissue injection. In some further embodiments, the instant pharmaceutical compositions can be administered by bolus injection, e.g., subcutaneous bolus injection, intramuscular bolus injection, intradermal bolus injection and the like. In one embodiment, the administration is via local infiltration to a surgical site to provide local analgesia. In another embodiment, the administration is via interscalene brachial plexus nerve block or femoral nerve block to provide regional analgesia. For pediatric subjects between the age of 6 and 17 years old, the administration of bupivacaine MVL composition described herein may be weight based, at about 4 mg/kg and up to 233 mg of bupivacaine.

Administration of the instant bupivacaine MVL composition may be accomplished using standard methods and devices, e.g., pens, injector systems, needle and syringe, a subcutaneous injection port delivery system, catheters, and the like. The administration of the bupivacaine MVLs composition may be used in conjunction with Pacira's handheld cryoanalgesia device.

### Pharmaceutical Compositions

In some embodiments, the composition comprising bupivacaine MVLs is a pharmaceutical formulation includes a pharmaceutically acceptable carrier. Effective injectable bupivacaine MVLs compositions is in a liquid suspension form. Such injectable suspension compositions require a liquid suspending medium, with or without adjuvants, as a vehicle. The suspending medium can be, for example, aqueous solutions of sodium chloride (i.e., saline solution), dextrose, sucrose, polyvinylpyrrolidone, polyethylene glycol, a pH modifying agent described herein, or combinations of the above. In some embodiments, the suspending medium of bupivacaine MVLs is a saline solution, optionally contain a tonicity agent such as dextrose and/or a pH modifying agent such as lysine.

Suitable physiologically acceptable storage solution components are used to keep the compound suspended in suspension compositions. The storage solution components can be chosen from thickeners such as carboxymethylcellulose, polyvinylpyrrolidone, gelatin and the alginates. Many surfactants are also useful as suspending agents. The suspending medium could also contain lecithin, alkylphenol polyethylene oxide adducts, naphthalenesulfonates, alkylbenzenesulfonates, or the polyoxyethylene sorbitan esters. In some embodiments, the bupivacaine MVL composition is free or substantially free of any additive of preservatives.

In any embodiments of the composition of bupivacaine encapsulated MVLs described herein, the composition may be a pharmaceutical composition suitable for human administration. In further embodiments, the composition may be an aqueous suspension of bupivacaine encapsulated MVL particles.

### EXAMPLES

The following examples, including experiments and results achieved, are provided for illustrative purposes only and are not to be construed as limiting the present application.

### Example 1: Lipid hydrolysis analysis based on erucic acid assay

In this example, the lipid stability of three batches (Batch No. 1, 2 and 3 in Tables 1A and 1B) of bupivacaine MVLs aqueous suspension prepared by the new process described herein and were compared to ten reference samples of bupivacaine MVLs aqueous suspension prepared by the current commercial process. DEPC hydrolysis byproduct erucic acid was used as the marker to measure the stability of the lipid membranes of the MVL particles. All the samples were incubated at 25°C for 1 month, 2 months, 3 months and 6 months. The pH of the supernatant of each sample (i.e., the external pH of the bupivacaine MVL composition) was also tested at each time point and summarized in Table 1B. Erucic acid was detected using HPLC and the erucic acid concentration in the sample was calculated based on the HPLC peak area and the standard curve.

**Table 1A. Erucic acid concentration in the bupivacaine MVLs as a functional of time**

| | **Erucic acid concentration (µg/mL)** | | | |
|---|---|---|---|---|
| **Batch** | 1 month | 2 months | 3 months | 6 months |
| 1 | 22 | 36 | 54 | 99 |
| 2 | 23 | 38 | 51 | 99 |
| 3 | 23 | 38 | 54 | 98 |
| Average | 22.7 | 37.3 | 53.0 | 98.7 |
| % RSD | 2.5 | 3.1 | 3.3 | 0.6 |

| **Reference samples** | 1 month | 2 months | 3 months | 6 months |
|---|---|---|---|---|
| Average | n/a | 38.7 | 55.4 | 113.1 |
| % RSD | n/a | 24.3 | 15.0 | 4.2 |

**Table 1B. External pH of bupivacaine MVLs compositions as a functional of time**

| | **External pH** | | | | |
|---|---|---|---|---|---|
| **Batch** | 0 month | 1 months | 2 months | 3 months | 6 months |
| 1 | 7.4 | 7.2 | 7.1 | 6.9 | 6.5 |
| 2 | 7.4 | 7.1 | 7.1 | 6.9 | 6.5 |
| 3 | 7.3 | 7.1 | 7.1 | 6.9 | 6.5 |
| Average | 7.4 | 7.1 | 7.1 | 6.9 | 6.5 |
| % RSD | 0.8 | 0.8 | 0.0 | 0.0 | 0.0 |

| **Ref. Samples** | 0 month | 1 months | 2 months | 3 months | 6 months |
|---|---|---|---|---|---|
| Average | 7.1 | 7.1 | 6.9 | 6.8 | 6.5 |
| % RSD | 1.4 | 1.6 | 1.5 | 0.8 | 0.6 |

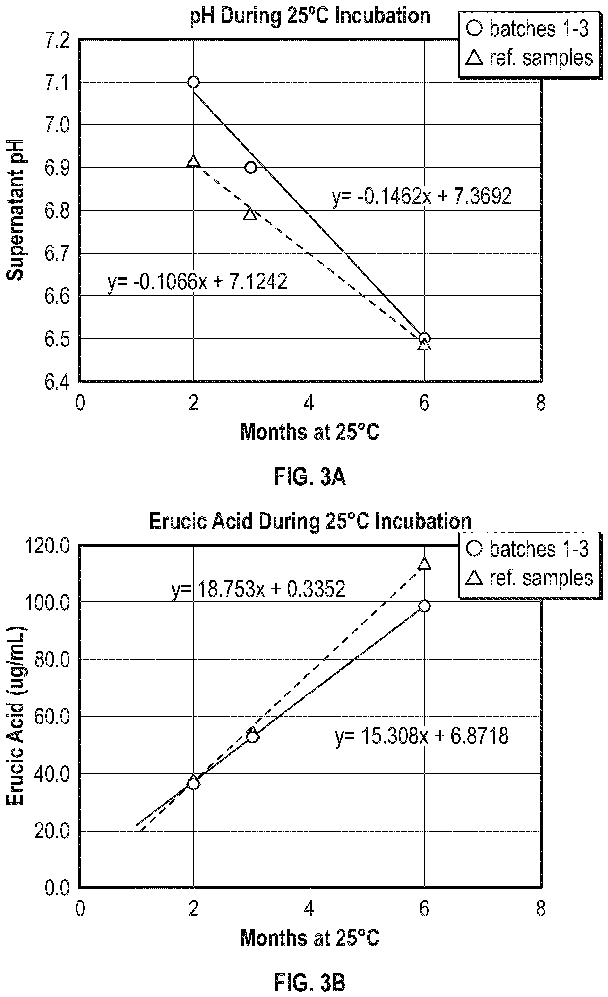

FIG. 3A is a line chart showing supernatant pH as a function of incubation time of the bupivacaine-MVL compositions prepared by the new process described herein as compared to those prepared by the existing commercial process. It is known that during incubation at 25°C, the Exparel^{®} product pH normally decreases slightly. During the six months period, the pH of bupivacaine MVL compositions prepared by the present process described herein decreased 37% faster than those prepared by the existing process, but still within the required 5.8-7.4 pH range.

FIG. 3B is a line chart showing erucic acid concentration as a function of incubation time at 25°C of the bupivacaine-MVL compositions prepared by the new process described herein as compared to those prepared by the existing commercial process. It was observed that the rate of lipid hydrolysis was 18% lower in the bupivacaine-MVL compositions prepared by the new process.

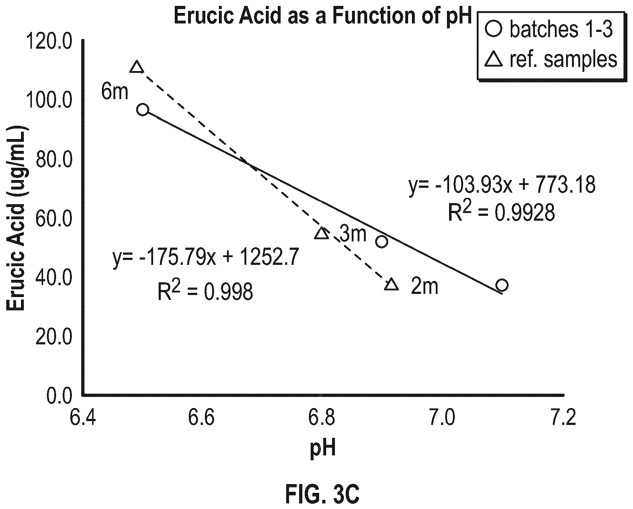

FIG. 3C is a line chart showing erucic acid concentration as a function of supernatant pH at 25°C. Typically, decreases in pH can both catalyze, and be a consequence of lipid hydrolysis. Therefore, a more rapid pH decline would normally be associated with a more rapid increase in erucic acid concentration. However, the slope for rate of change of erucic acid concentration as a function of (decreasing) pH was actually flatter for the bupivacaine-MVL compositions prepared by the new process as compared to those prepared by the existing commercial process. The improved lipid stability (as indicated by the erucic acid concentration) observed in the bupivacaine MVLs prepared by the presently described process was surprisingly unexpected.

### Example 2: Measurement of lysine and dextrose concentrations in bupivacaine MVLs

In this experiment, the lysine and dextrose concentration were measured in three batches (Batch No. 1, 2 and 3 in Tables 2A and 2B) of bupivacaine MVLs aqueous suspension prepared by the new process described herein and compared to several reference samples of bupivacaine MVLs aqueous suspension prepared by the current commercial process.

**Table 2A. Lysine and dextrose concentrations in bupivacaine MVL compositions**

| | Total Suspension | | MVL particles | |
|---|---|---|---|---|
| Batch | Dextrose (mg/mL) | Lysine (mg/mL) | Dextrose (mg/mL) | Lysine (mg/mL) |
| 1 | 2.19 | 0.12 | 1.32 | 0.031 |
| 2 | 2.17 | 0.12 | 1.30 | 0.030 |
| 3 | 2.15 | 0.12 | 1.25 | 0.032 |
| Average | 2.17 | 0.12 | 1.29 | 0.031 |
| Avg. Ref. Samples | 1.86 | 0.11 | 1.14 | 0.024 |
| Avg./Avg. Ref. Samples | 1.17 | 1.08 | 1.13 | 1.29 |

**Table 2B. External and internal pH in bupivacaine MVL compositions**

| Batch | Internal pH | Avg Internal pH | Time 0 Sup pH |
|---|---|---|---|
| 1 | 5.49 | 5.50 | 7.4 |
| 2 | 5.51 | | |
| 3 | 5.50 | | |
| Ref. samples | | 5.38 | 7.1 |

It was observed that the total suspensions and bupivacaine MVL particles prepared by the present process contained approximately 17% and 13% more dextrose, respectively, than those samples prepared by the existing commercial process. In addition, the lysine concentration was 8% and 29% more in those samples prepared by the present process. In addition, the internal and external pH of the bupivacaine MVL compositions were also measured. The higher internal pH of the bupivacaine MVL particles prepared by the present process may be attributable to the higher lysine concentration inside the MVL particles. As discussed above, the slight increase in MVL internal pH may also contribute to the stability of the lipid membranes.

## Claims

1. A composition of bupivacaine encapsulated multivesicular liposomes (MVLs), comprising:
bupivacaine residing inside a plurality of internal aqueous chambers of the MVLs separated by lipid membranes, wherein the lipid membranes comprise 1, 2-dierucoylphosphatidylcholine (DEPC), 1, 2-dipalmitoyl-sn-glycero-3 phospho-rac-(1-glycerol) (DPPG), and at least one neutral lipid; and
an aqueous medium in which the bupivacaine encapsulated MVLs are suspended;
wherein an erucic acid concentration in the composition is 23 µg/mL or less after the composition is stored at 25°C for one month and less than 109µg/mL after the composition is stored at 25°C for six months; and
wherein the plurality of internal aqueous chambers of the MVLs comprise lysine, and the encapsulated lysine concentration in the composition is at least 0.03 mg/mL;
wherein the composition is prepared by a process comprising:
(a) mixing a first aqueous solution comprising phosphoric acid with a volatile water-immiscible solvent solution to form a water-in-oil first emulsion, wherein the volatile water-immiscible solvent solution comprises bupivacaine, 1, 2-dierucoylphosphatidylcholine (DEPC), 1, 2-dipalmitoyl-sn-glycero-3 phospho-rac-(1-glycerol) (DPPG), and at least one neutral lipid;
(b) mixing the water-in-oil first emulsion with a second aqueous solution comprising lysine and dextrose to form a water-in-oil-in-water second emulsion;
(c) removing the volatile water-immiscible solvent from the water-in-oil-in-water second emulsion to form a first aqueous suspension of bupivacaine encapsulated multivesicular liposomes having a first volume;
(d) reducing the first volume of the first aqueous suspension of bupivacaine encapsulated multivesicular liposomes by microfiltration to provide a second aqueous suspension of bupivacaine encapsulated multivesicular liposomes having a second volume;
(e) exchanging the aqueous supernatant of the second aqueous suspension with a saline solution by diafiltration to provide a third aqueous suspension of bupivacaine encapsulated multivesicular liposomes having a third volume; and
(f) further reducing the third volume of the third aqueous suspension by microfiltration to provide a final aqueous suspension of bupivacaine encapsulated multivesicular liposomes having a target concentration of bupivacaine;
wherein all steps are carried out under aseptic conditions.

2. The composition of claim 1, wherein the encapsulated lysine concentration in the composition is 0.03 mg/mL.

3. The composition of claim 1 or 2, wherein the erucic acid concentration in the composition is 99 µg/mL or less after the composition is stored at 25°C for six months.

4. The composition of any one of claims 1 to 3, wherein the lipid membranes further comprise cholesterol and tricaprylin.

5. The composition of any one of claims 1 to 4, wherein the plurality of internal aqueous chambers of the MVLs has an initial pH of 5.5.

6. The composition of any one of claims 1 to 5, wherein the plurality of internal aqueous chambers of the MVLs comprise dextrose, and the encapsulated dextrose concentration in the composition is 1.25 mg/mL to 1.32 mg/mL.

7. The composition of any one of claims 1 to 6, wherein the bupivacaine concentration in the composition is from 11.3 mg/mL to 17.0 mg/mL.

8. The composition of claim 7, wherein the bupivacaine concentration in the composition is 13.3 mg/mL.

9. The composition of any one of claims 1 to 8, wherein the percent packed particle volume (% PPV) of the bupivacaine encapsulated multivesicular liposomes in the composition is 35% to 40%.

10. A composition according to any one of claims 1 to 9 for use in a method of treating or ameliorating pain in a subject.

11. The composition for use of claim 10, wherein the composition is for administration via local infiltration to a surgical site to provide local analgesia, or for administration via interscalene brachial plexus nerve block or femoral nerve block to provide regional analgesia.

12. The composition for use of claim 10 or 11, comprising a single dose of 10 mL or 20 mL of the composition having a bupivacaine concentration of 13.3 mg/mL.

13. A process for preparing bupivacaine encapsulated multivesicular liposomes, the process comprising:
(a) mixing a first aqueous solution comprising phosphoric acid with a volatile water-immiscible solvent solution to form a water-in-oil first emulsion, wherein the volatile water-immiscible solvent solution comprises bupivacaine, 1, 2-dierucoylphosphatidylcholine (DEPC), 1, 2-dipalmitoyl-sn-glycero-3 phospho-rac-(1-glycerol) (DPPG), and at least one neutral lipid;
(b) mixing the water-in-oil first emulsion with a second aqueous solution comprising lysine and dextrose to form a water-in-oil-in-water second emulsion;
(c) removing the volatile water-immiscible solvent from the water-in-oil-in-water second emulsion to form a first aqueous suspension of bupivacaine encapsulated multivesicular liposomes having a first volume;
(d) reducing the first volume of the first aqueous suspension of bupivacaine encapsulated multivesicular liposomes by microfiltration to provide a second aqueous suspension of bupivacaine encapsulated multivesicular liposomes having a second volume;
(e) exchanging the aqueous supernatant of the second aqueous suspension with a saline solution by diafiltration to provide a third aqueous suspension of bupivacaine encapsulated multivesicular liposomes having a third volume; and
(f) further reducing the third volume of the third aqueous suspension by microfiltration to provide a final aqueous suspension of bupivacaine encapsulated multivesicular liposomes having a target concentration of bupivacaine;
wherein all steps are carried out under aseptic conditions, and wherein erucic acid concentration in the final aqueous suspension of bupivacaine encapsulated multivesicular liposomes is 23 µg/mL or less after the final aqueous suspension is stored at 25°C for one month and less than 109 µg/mL after the final aqueous suspension is stored at 25°C for six months; and wherein the encapsulated lysine concentration in the final aqueous suspension of bupivacaine encapsulated multivesicular liposomes is at least 0.03 mg/mL.

14. The process of claim 13, wherein the encapsulated lysine concentration in the final aqueous suspension of bupivacaine encapsulated multivesicular liposomes is 0.03 mg/mL.

15. The process of claim 13 or 14, wherein erucic acid concentration in the final aqueous suspension of bupivacaine encapsulated multivesicular liposomes is 99 µg/mL or less after the final aqueous suspension is stored at 25°C for six months.

16. The process of any one of claims 13 to 15, wherein the mixing of step (a) is performed using a mixer at a high shear speed from about 1100 rpm to about 1200 rpm.

17. The process of any one of claims 13 to 16, wherein the mixing of step (b) is performed using two mixers at a low shear speed from about 450 rpm to about 510 rpm.

18. The process of any one of claims 13 to 17, wherein the target concentration of bupivacaine is from 11.3 mg/mL to 17 mg/mL.

19. The process of any one of claims 13 to 18, wherein the internal pH of the bupivacaine encapsulated multivesicular liposomes in the final aqueous suspension is 5.5.

20. The process of any one of claims 13 to 19, wherein the encapsulated dextrose concentration in the final aqueous suspension of bupivacaine encapsulated multivesicular liposomes is 1.25 mg/mL to 1.32 mg/mL.

## Patentansprüche

1. Zusammensetzung aus Bupivacain-gekapselten multivesikulären Liposomen (MVLs), umfassend:
Bupivacain, das sich in einer Vielzahl von inneren wässrigen Kammern der MVLs befindet, die durch Lipidmembranen voneinander getrennt sind, wobei die Lipidmembranen 1,2-Dierucoylphosphatidylcholin (DEPC), 1,2-Dipalmitoyl-sn-glycero-3-phospho-rac-(1-glycerol) (DPPG) und mindestens ein neutrales Lipid umfassen, und ein wässriges Medium, in dem die Bupivacain-gekapselten MVLs in Suspension gehalten werden,
wobei die Erucasäurekonzentration in der Zusammensetzung nach einer einmonatigen Lagerung bei 25 °C 23 µg/ml oder weniger und nach einer sechsmonatigen Lagerung bei 25 °C weniger als 109 µg/ml beträgt, und
wobei die Vielzahl der inneren wässrigen Kammern der MVLs Lysin umfassen und die Konzentration des eingekapselten Lysins in der Zusammensetzung mindestens 0,03 mg/ml beträgt,
wobei die Zusammensetzung durch ein Verfahren hergestellt wird, das umfasst:
(a) Mischen einer ersten wässrigen Lösung, umfassend Phosphorsäure, mit einer flüchtigen, mit Wasser nicht mischbaren Lösungsmittellösung, um eine erste Wasser-in-Öl-Emulsion zu bilden, wobei die flüchtige, mit Wasser nicht mischbare Lösungsmittellösung Bupivacain, 1,2-Dierucoylphosphatidylcholin (DEPC), 1,2-Dipalmitoyl-sn-glycero-3-phospho-rac-(1-glycerol) (DPPG) und mindestens ein neutrales Lipid umfasst;
(b) Mischen der ersten Wasser-in-Öl-Emulsion mit einer zweiten wässrigen Lösung, die Lysin und Dextrose umfasst, um eine zweite Wasser-in-Öl-in-Wasser-Emulsion zu bilden;
(c) Entfernen des flüchtigen, mit Wasser nicht mischbaren Lösungsmittels aus der zweiten Emulsion vom Typ Wasser-in-Öl-in-Wasser, um eine erste wässrige Suspension aus Bupivacain-gekapselten multivesikulären Liposomen mit einem ersten Volumen zu bilden;
(d) Reduzieren des ersten Volumens der ersten wässrigen Suspension von Bupivacain-gekapselten multivesikulären Liposomen durch Mikrofiltration, um eine zweite wässrige Suspension von Bupivacain-gekapselten multivesikulären Liposomen mit einem zweiten Volumen bereitzustellen;
(e) Austauschen des wässrigen Überstands der zweiten wässrigen Suspension durch eine Salzlösung mittels Diafiltration, um eine dritte wässrige Suspension von Bupivacain-gekapselten multivesikulären Liposomen mit einem dritten Volumen bereitzustellen, und
(f) weiteres Reduzieren des dritten Volumens der dritten wässrigen Suspension durch Mikrofiltration, um eine endgültige wässrige Suspension von Bupivacain-gekapselten multivesikulären Liposomen mit einer Zielkonzentration von Bupivacain bereitzustellen;
wobei alle Schritte unter aseptischen Bedingungen durchgeführt werden.

2. Zusammensetzung nach Anspruch 1, wobei die Konzentration an eingekapseltem Lysin in der Zusammensetzung 0,03 mg/ml beträgt.

3. Zusammensetzung nach Anspruch 1 oder 2, wobei die Erucasäurekonzentration in der Zusammensetzung nach sechsmonatiger Lagerung bei 25 °C 99 µg/ml oder weniger beträgt.

4. Zusammensetzung nach einem der Ansprüche 1 bis 3, wobei die Lipidmembranen zusätzlich Cholesterin und Tricaprylin umfassen.

5. Zusammensetzung nach einem der Ansprüche 1 bis 4, wobei die Vielzahl der inneren wässrigen Kammern der MVLs einen anfänglichen pH-Wert von 5,5 aufweist.

6. Zusammensetzung nach einem der Ansprüche 1 bis 5, wobei die Vielzahl der inneren wässrigen Kammern der MVLs Dextrose umfasst und die Konzentration der eingekapselten Dextrose in der Zusammensetzung 1,25 mg/ml bis 1,32 mg/ml beträgt.

7. Zusammensetzung nach einem der Ansprüche 1 bis 6, wobei die Bupivacainkonzentration in der Zusammensetzung zwischen 11,3 mg/ml und 17,0 mg/ml beträgt.

8. Zusammensetzung nach Anspruch 7, wobei die Bupivacainkonzentration in der Zusammensetzung 13,3 mg/ml beträgt.

9. Zusammensetzung nach einem der Ansprüche 1 bis 8, wobei das prozentuale gepackte Partikelvolumen (% PPV) der Bupivacain-gekapselten multivesikulären Liposomen in der Zusammensetzung 35 % bis 40 % beträgt.

10. Zusammensetzung nach einem der Ansprüche 1 bis 9 zur Verwendung in einem Verfahren zur Behandlung oder Linderung von Schmerzen bei einer Person.

11. Zusammensetzung zur Verwendung nach Anspruch 10, wobei die Zusammensetzung zur Verabreichung durch lokale Infiltration an einer Operationsstelle zur lokalen Anästhesie oder zur Verabreichung durch interskalenäre Plexus-brachialis-Blockade oder Femoralis-Blockade zur regionalen Anästhesie bestimmt ist.

12. Zusammensetzung zur Verwendung nach Anspruch 10 oder 11, umfassend eine Einzeldosis von 10 ml oder 20 ml der Zusammensetzung mit einer Bupivacainkonzentration von 13,3 mg/ml.

13. Verfahren zur Herstellung von Bupivacain-gekapselten multivesikulären Liposomen, wobei das Verfahren umfasst:
(a) Mischen einer ersten wässrigen Lösung, umfassend Phosphorsäure, mit einer flüchtigen, mit Wasser nicht mischbaren Lösungsmittellösung, um eine erste Wasser-in-Öl-Emulsion zu bilden, wobei die flüchtige, mit Wasser nicht mischbare Lösungsmittellösung Bupivacain, 1,2-Dierucoylphosphatidylcholin (DEPC), 1,2-Dipalmitoyl-sn-glycero-3-phospho-rac-(1-glycerol) (DPPG) und mindestens ein neutrales Lipid umfasst;
(b) Mischen der ersten Wasser-in-Öl-Emulsion mit einer zweiten wässrigen Lösung, die Lysin und Dextrose umfasst, um eine zweite Wasser-in-Öl-in-Wasser-Emulsion zu bilden;
(c) Entfernen des flüchtigen, mit Wasser nicht mischbaren Lösungsmittels aus der zweiten Emulsion vom Typ Wasser-in-Öl-in-Wasser, um eine erste wässrige Suspension aus Bupivacain-gekapselten multivesikulären Liposomen mit einem ersten Volumen zu bilden;
(d) Reduzieren des ersten Volumens der ersten wässrigen Suspension von Bupivacain-gekapselten multivesikulären Liposomen durch Mikrofiltration, um eine zweite wässrige Suspension von Bupivacain-gekapselten multivesikulären Liposomen mit einem zweiten Volumen bereitzustellen;
(e) Austauschen des wässrigen Überstands der zweiten wässrigen Suspension durch eine Salzlösung mittels Diafiltration, um eine dritte wässrige Suspension von Bupivacain-gekapselten multivesikulären Liposomen mit einem dritten Volumen bereitzustellen, und
(f) weiteres Reduzieren des dritten Volumens der dritten wässrigen Suspension durch Mikrofiltration, um eine endgültige wässrige Suspension von Bupivacain-gekapselten multivesikulären Liposomen mit einer Zielkonzentration von Bupivacain bereitzustellen;
wobei alle Schritte unter aseptischen Bedingungen durchgeführt werden und wobei die Erucasäurekonzentration in der endgültigen wässrigen Suspension der Bupivacain-gekapselten multivesikulären Liposomen 23 µg/ml oder weniger beträgt, nachdem die endgültige wässrige Suspension einen Monat lang bei 25 °C gelagert wurde, und weniger als 109 µg/ml beträgt, nachdem die endgültige wässrige Suspension sechs Monate lang bei 25 °C gelagert wurde; und wobei die Lysinkonzentration in der endgültigen wässrigen Suspension der Bupivacain-gekapselten multivesikulären Liposomen mindestens 0,03 mg/ml beträgt.

14. Verfahren nach Anspruch 13, wobei die Konzentration des eingekapselten Lysins in der endgültigen wässrigen Suspension von Bupivacain-gekapselten multivesikulären Liposomen 0,03 mg/ml beträgt.

15. Verfahren nach Anspruch 13 oder 14, wobei die Erucasäurekonzentration in der endgültigen wässrigen Suspension von Bupivacain-gekapselten multivesikulären Liposomen 99 µg/ml oder weniger beträgt, nachdem die endgültige wässrige Suspension sechs Monate lang bei 25 °C gelagert wurde.

16. Verfahren nach einem der Ansprüche 13 bis 15, wobei das Mischen in Schritt (a) unter Verwendung eines Mischers mit einer hohen Schergeschwindigkeit von etwa 1100 U/min bis etwa 1200 U/min durchgeführt wird.

17. Verfahren nach einem der Ansprüche 13 bis 16, wobei das Mischen in Schritt (b) unter Verwendung von zwei Mischern bei einer niedrigen Schergeschwindigkeit von etwa 450 U/min bis etwa 510 U/min durchgeführt wird.

18. Verfahren nach einem der Ansprüche 13 bis 17, wobei die Zielkonzentration von Bupivacain zwischen 11,3 mg/ml und 17 mg/ml beträgt.

19. Verfahren nach einem der Ansprüche 13 bis 18, wobei der interne pH-Wert der Bupivacain-gekapselten multivesikulären Liposomen in der endgültigen wässrigen Suspension 5,5 beträgt.

20. Verfahren nach einem der Ansprüche 13 bis 19, wobei die Konzentration der eingekapselten Dextrose in der endgültigen wässrigen Suspension von Bupivacain-gekapselten multivesikulären Liposomen 1,25 mg/ml bis 1,32 mg/ml beträgt.

## Revendications

1. Composition de liposomes multivésiculaires (MVL) encapsulés dans de la bupivacaïne, comprenant :
de la bupivacaïne résidant à l'intérieur d'une pluralité de chambres aqueuses internes des MVL séparées par des membranes lipidiques, dans laquelle les membranes lipidiques comprennent de la 1,2-diérucoylphosphatidylcholine (DEPC), du 1,2-dipalmitoyl-sn-glycéro-3 phospho-rac-(1-glycérol) (DPPG), et au moins un lipide neutre ; et
un milieu aqueux dans lequel sont mis en suspension les MVL encapsulés dans de la bupivacaïne ;
dans laquelle une concentration d'acide érucique dans la composition est de 23 µg/mL ou moins après que la composition est conservée à 25 °C pendant un mois et moins de 109 µg/mL après que la composition est conservée à 25 °C pendant six mois ; et
dans laquelle la pluralité de chambres aqueuses internes des MVL comprennent de la lysine, et la concentration de lysine encapsulée dans la composition est d'au moins 0,03 mg/mL ;
dans laquelle la composition est préparée par un procédé comprenant :
(a) le mélange d'une première solution aqueuse comprenant de l'acide phosphorique avec une solution de solvant non miscible à l'eau volatil pour former une première émulsion eau dans huile, dans lequel la solution de solvant non miscible à l'eau volatil comprend de la bupivacaïne, de la 1,2-diérucoylphosphatidylcholine (DEPC), du 1,2-dipalmitoyl-sn-glycéro-3 phospho-rac-(1-glycérol) (DPPG), et au moins un lipide neutre ;
(b) le mélange de la première émulsion eau dans huile avec une seconde solution aqueuse comprenant de la lysine et du dextrose pour former une seconde émulsion eau dans huile dans eau ;
(c) l'élimination du solvant non miscible à l'eau volatil de la seconde émulsion eau dans huile dans eau pour former une première suspension aqueuse de liposomes multivésiculaires encapsulés dans de la bupivacaïne ayant un premier volume ;
(d) la réduction du premier volume de la première suspension aqueuse de liposomes multivésiculaires encapsulés dans de la bupivacaïne par microfiltration pour fournir une deuxième suspension aqueuse de liposomes multivésiculaires encapsulés dans de la bupivacaïne ayant un second volume ;
(e) l'échange du surnageant aqueux de la deuxième suspension aqueuse avec une solution saline par diafiltration pour fournir une troisième suspension aqueuse de liposomes multivésiculaires encapsulés dans de la bupivacaïne ayant un troisième volume ; et
(f) la réduction supplémentaire du troisième volume de la troisième suspension aqueuse par microfiltration pour fournir une suspension aqueuse finale de liposomes multivésiculaires encapsulés dans de la bupivacaïne ayant une concentration cible de bupivacaïne ;
dans lequel toutes les étapes sont effectuées dans des conditions aseptiques.

2. Composition selon la revendication 1, dans laquelle la concentration de lysine encapsulée dans la composition est de 0,03 mg/mL.

3. Composition selon la revendication 1 ou 2, dans laquelle la concentration d'acide érucique dans la composition est de 99 µg/mL ou moins après que la composition est conservée à 25 °C pendant six mois.

4. Composition selon l'une quelconque des revendications 1 à 3, dans laquelle les membranes lipidiques comprennent en outre du cholestérol et de la tricapryline.

5. Composition selon l'une quelconque des revendications 1 à 4, dans laquelle la pluralité de chambres aqueuses internes des MVL a un pH initial de 5,5.

6. Composition selon l'une quelconque des revendications 1 à 5, dans laquelle la pluralité de chambres aqueuses internes des MVL comprend du dextrose, et la concentration de dextrose encapsulé dans la composition est de 1,25 mg/mL à 1,32 mg/mL.

7. Composition selon l'une quelconque des revendications 1 à 6, dans laquelle la concentration de bupivacaïne dans la composition est de 11,3 mg/mL à 17,0 mg/mL.

8. Composition selon la revendication 7, dans laquelle la concentration de bupivacaïne dans la composition est de 13,3 mg/mL.

9. Composition selon l'une quelconque des revendications 1 à 8, dans laquelle le pourcentage de volume de particules tassées (% PPV) des liposomes multivésiculaires encapsulés dans de la bupivacaïne dans la composition est de 35 % à 40 %.

10. Composition selon l'une quelconque des revendications 1 à 9, destinée à être utilisée dans un procédé de traitement ou d'amélioration de la douleur chez un sujet.

11. Composition destinée à être utilisée selon la revendication 10, dans laquelle la composition est destinée à être administrée par infiltration locale à un site chirurgical pour fournir une analgésie locale, ou à être administrée via un bloc nerveux du plexus brachial interscalène ou un bloc nerveux fémoral pour fournir une analgésie régionale.

12. Composition destinée à être utilisée selon la revendication 10 ou 11, comprenant une dose unique de 10 mL ou 20 mL de la composition ayant une concentration de bupivacaïne de 13,3 mg/mL.

13. Procédé de préparation de liposomes multivésiculaires encapsulés dans de la bupivacaïne, le procédé comprenant :
(a) le mélange d'une première solution aqueuse comprenant de l'acide phosphorique avec une solution de solvant non miscible à l'eau volatil pour former une première émulsion eau dans huile, dans lequel la solution de solvant non miscible à l'eau volatil comprend de la bupivacaïne, de la 1,2-diérucoylphosphatidylcholine (DEPC), du 1,2-dipalmitoyl-sn-glycéro-3 phospho-rac-(1-glycérol) (DPPG), et au moins un lipide neutre ;
(b) le mélange de la première émulsion eau dans huile avec une seconde solution aqueuse comprenant de la lysine et du dextrose pour former une seconde émulsion eau dans huile dans eau ;
(c) l'élimination du solvant non miscible à l'eau volatil de la seconde émulsion eau dans huile dans eau pour former une première suspension aqueuse de liposomes multivésiculaires encapsulés dans de la bupivacaïne ayant un premier volume ;
(d) la réduction du premier volume de la première suspension aqueuse de liposomes multivésiculaires encapsulés dans de la bupivacaïne par microfiltration pour fournir une deuxième suspension aqueuse de liposomes multivésiculaires encapsulés dans de la bupivacaïne ayant un second volume ;
(e) l'échange du surnageant aqueux de la deuxième suspension aqueuse avec une solution saline par diafiltration pour fournir une troisième suspension aqueuse de liposomes multivésiculaires encapsulés dans de la bupivacaïne ayant un troisième volume ; et
(f) la réduction supplémentaire du troisième volume de la troisième suspension aqueuse par microfiltration pour fournir une suspension aqueuse finale de liposomes multivésiculaires encapsulés dans de la bupivacaïne ayant une concentration cible de bupivacaïne ;
dans lequel toutes les étapes sont effectuées dans des conditions aseptiques, et dans lequel la concentration d'acide érucique dans la suspension aqueuse finale de liposomes multivésiculaires encapsulés dans de la bupivacaïne est de 23 µg/mL ou moins après que la suspension aqueuse finale est conservée à 25 °C pendant un mois et inférieure à 109 µg/mL après que la suspension aqueuse finale est conservée à 25 °C pendant six mois ; et dans lequel la concentration de lysine encapsulée dans la suspension aqueuse finale de liposomes multivésiculaires encapsulés dans de la bupivacaïne est d'au moins 0,03 mg/mL.

14. Procédé selon la revendication 13, dans lequel la concentration de lysine encapsulée dans la suspension aqueuse finale de liposomes multivésiculaires encapsulés dans de la bupivacaïne est de 0,03 mg/mL.

15. Procédé selon la revendication 13 ou 14, dans lequel la concentration d'acide érucique dans la suspension aqueuse finale de liposomes multivésiculaires encapsulés dans de la bupivacaïne est de 99 µg/mL ou moins après que la suspension aqueuse finale est conservée à 25 °C pendant six mois.

16. Procédé selon l'une quelconque des revendications 13 à 15, dans lequel le mélange de l'étape (a) est réalisé en utilisant un mélangeur à une vitesse de cisaillement élevée d'environ 1 100 tpm à environ 1 200 tpm.

17. Procédé selon l'une quelconque des revendications 13 à 16, dans lequel le mélange de l'étape (b) est réalisé en utilisant deux mélangeurs à une vitesse de cisaillement faible d'environ 450 tpm à environ 510 tpm.

18. Procédé selon l'une quelconque des revendications 13 à 17, dans lequel la concentration cible de bupivacaïne est de 11,3 mg/mL à 17 mg/mL.

19. Procédé selon l'une quelconque des revendications 13 à 18, dans lequel le pH interne des liposomes multivésiculaires encapsulés dans de la bupivacaïne dans la suspension aqueuse finale est de 5,5.

20. Procédé selon l'une quelconque des revendications 13 à 19, dans lequel la concentration de dextrose encapsulé dans la suspension aqueuse finale de liposomes multivésiculaires encapsulés dans de la bupivacaïne est de 1,25 mg/mL à 1,32 mg/mL.
